# EUROPÄISCHE PATENTSCHRIFT

(11) **EP 2 110 126 B1**
(45) Veröffentlichungstag und Bekanntmachung des Hinweises auf die Patenterteilung: **09.11.2011**
(21) Anmeldenummer: 08007640.9
(22) Anmeldetag: 18.04.2008
(51) Int. Cl.: A61K 9/72, A61K 31/07, A61K 31/135, A61K 31/137, A61K 31/192, A61K 31/196, A61K 31/355, A61K 31/513, C07C 19/08

(54) **Inhalative und instillative Verwendung von semifluorierten Alkanen als Wirkstoffträger im intrapulmonalen Bereich**
Inhalative and instillative use of semi-fluorised alkanes as active ingredient carriers in the intrapulmonary area
Utilisation pour inhalation et instillation d'alcanes semifluorées comme support de matière active dans la zone intrapulmonaire

(43) Veröffentlichungstag der Anmeldung: 21.10.2009
(73) Patentinhaber: Novaliq GmbH, 69120 Heidelberg (DE)
(72) Erfinder: Meinert, Hasso, Prof. Dr., 89231 Neu-Ulm (DE)
(74) Vertreter: Leissler-Gerstl, Gabriele

(56) Entgegenhaltungen:
- EP-B1- 0 965 334
- WO-A-97/38579
- WO-A-2005/099718
- WO-A-2007/059968
- LEHMLER, H-J ET AL.: "Liquid ventilation - A new way to deliver drugs to diseased lungs" CHEMTECH, [Online] Bd. 29, Nr. 10, Oktober 1999 (1999-10), Seiten 7-12, XP002494765 Gefunden im Internet: URL:http://pubs.acs.org/hotartcl/chemtech/ 99/oct/lungs.html> [gefunden am 2008-09-05]
- DATABASE MEDLINE [Online] US NATIONAL LIBRARY OF MEDICINE (NLM), BETHESDA, MD, US; 3 July 2008 (2008-07-03), BRONIATOWSKI MARCIN ET AL: "Two-dimensional miscibility studies of alamethicin and selected film-forming molecules.", Database accession no. NLM18543870 & THE JOURNAL OF PHYSICAL CHEMISTRY. B 3 JUL 2008, vol. 112, no. 26, 3 July 2008 (2008-07-03) , pages 7762-7770, ISSN: 1520-6106
- KH Bauer, KH Frömming, C. Führer (Ed.): "Pharmazeutische Technologie", 1986, Georg Thieme Verlag, Stuttgart, DE pages 186-189-236-237,

## Beschreibung

Zunehmend wird die Gesundheit der Menschen durch Industrie-Stäube und -Abgase, Feinstäube, chronische Bronchitis und Lungenemphysem beeinträchtigt. Das Krankheitsbild eines schweren, akuten Lungenversagens ist durch eine anhaltend ausgeprägte Störung des pulmonalen Gasaustauschs, einen extremen Abfall der Compliance des respiratorischen Systems und ein interstitielles und alveoläres Lungenödem charakterisiert. Die Letalität wird bis heute noch mit über 50% angegeben. Ein Grund hierfür beruht auf der sehr aggressiven maschinellen Beatmung, die zur Aufrechterhaltung annähernd normaler Blutgase erforderlich ist.

Als therapeutische Alternative wurden Perfluorcarbone "PFCs", vollständig fluorierte Kohlenstoff Verbindungen, mit einer hohen Löslichkeit für Sauerstoff, aber einer sehr hohen Dichte, zur vollständigen Flüssigkeitsbeatmung eingesetzt (1). Diese reine Flüssigkeitsbeatmung ist jedoch technisch schwer durchführbar. Daher wurde in den späten 90er Jahren die partielle Flüssigkeitsbeatmung (Partial Liquid Ventilation "PLV") mittels Perfluorcarbonen erstmals klinisch angewendet (2). Hierbei wird während normaler Gasbeatmung Perfluorcarbon bolusweise bis zu einem maximalen Volumen, entsprechend der funktionellen Residualkapazität, intratracheal instilliert. Durch die Beatmung wird die Flüssigkeit in der Lunge verteilt und die Standardgasbeatmung kann mit positiven Atemdrücken weitergeführt werden.

Als medizinische Hilfsmittel sind Semifluorierte Alkane "SFAs" zur vollständigen oder partiellen Liquid Ventilation bei chirurgischen Eingriffen, zur Entfaltung von Atelektase-Lungen oder kollabierten Lungen bekannt (3-5). Gemäß (6) können Wirkstoffe in Form von Mikrokugeln mittels Surfactants in Fluorcarbonen dispergiert und intratracheal appliziert werden. In (7) wird eine wässrige Emulsion von Phospholipiden in Fluorcarbonen vorgeschlagen, die, als Aerosol appliziert, die Fluidität des natürlichen Lungensurfactants verbessern soll.

WO 97/38579 beschreibt eine Emulsion enthaltend Perfluoro-n-octyl-decan, 1-Perfluoro-octyl-heptan, und Phospholipide als Lösungsmittel für Stickstoffmonoxid. Die Emulsion ist geeignet als medizinisches Hilfsmittel zum Transport von Stickstoffmonoxid in den Lungebereich eines Patienten.

Aus WO 2005/099718 ist es bekannt, die Fluidität des nativen Lungensurfactants zu erhöhen durch Bildung einer Emulsion aus einem Fluorkohlenstoff und Phospholipiden unter der Einwirkung des oberflächenspannungsreduzierenden Surfactants.

Weiterhin ist es aus WO 97/38579 bekannt, Stickoxid in Form einer Emulsion in die Lunge zu bringen.

Aufgabe der Erfindung ist es, ein medizinisches Hilfsmittel zu schaffen, mit dem ein Medikamententransport in Lungenbereiche und die Blutbahn eines Patienten erreicht wird.

Diese Aufgabe wird durch die Merkmale der Patentansprüche 1 - 11 gelöst. Die Unteransprüche geben vorteilhafte Weiterbildungen der Erfindung an.

Durch die Erfindung wird ein medizinisches Hilfsmittel zum direkten Transport wenigstens eines Medikaments in Lungenbereiche und die Blutbahn eines Patienten geschaffen, bei dem als Wirkstoffträger wenigstens ein semifluoriertes Alkan vorgesehen ist. Dabei kommt ein lineares semifluoriertes Alkan mit der allgemeinen Formel RFRH zur Anwendung.

Semifluorierte Alkane des Typs RFRH sind Verbindungen, die aus einem Perfluorcarbon-Segment "RF" und einem Hydrocarbon-Segment "RH" bestehen, wobei verzweigte, gesättigte Kohlenwasserstoff-Gruppe ist (3-5). Die Verbindungen F(CF2)ₙ(CH2)ₘH, mit n und m = 2 bis 20, sind flüssig, farblos, wasserunlöslich, physikalisch- chemisch und physiologisch inert. Die Siedepunkte korrelieren mit den Massenanteilen der RF- und RH-Segmente im Molekül (Tabb. 1, 2). SFAs haben Dichten von 1,1 bis 1,7 g/cm³, sehr niedrige Grenzflächen- und Oberflächen-Spannungen (ca. 45 bzw. 19 mN/m) und hohe Dampfdrücke (5 bis 760 Torr bei 25°C ). SFAs besitzen sehr hohe Gaslöslichkeiten und ein außerordentlich hohes Spreitungsvermögen, wobei Letzteres um ein Vielfaches höher als von PFCs ist (8, 9).

Die nichtsymmetrischen RFRHs sind amphiphile Verbindungen infolge des lipophoben RF- und lipophilen RH-Segmentes. Die Lipophilie steigt mit der Länge des RH-Teils und fällt umgekehrt mit zunehmendem RF-Anteil im Molekül. Gegenüber den unpolaren PFCs besitzen RFRHs auf Grund ihrer Lipophilie ein gutes Lösevermögen für Kohlenwasserstoffe und deren Derivate, somit auch für viele Wirkstoffe bzw. Medikamente. Dabei nimmt im RFRH mit steigendem RH-Anteil die Löslichkeit in organischen Verbindungen zu. (Im Anwendungsfall müssen die betreffenden Wirkstoffe in ihrer Basisform, also nicht als wasserlöslich gemachte Hydrochloride, Phosphate oder Alkalisalze eingesetzt werden).

Im Solvens SFA sind die Wirkstoffe in homgener Phase rein physikalisch, je nach Verteilungsgrad von < 100 nm, kolloid- bis molekulardispers gelöst.

Hieraus resultieren folgende vorzüglichen Eigenschaften der Erfindung:
- Sicherung des Gasaustausches zwischen der Alveolarluft und dem in den Lungenkapillaren fliessenden Blut,
- hohe Grenzflächenaktivität durch niedrige Grenzflächen-und Oberflächen-Spannungen,
- sehr hohes Spreitungsvermögen,
- Carrier auf Grund der Löslichkeiten von Kohlenwasserstoffen, deren Derivaten, wie Wirkstoffe und Medikamente.

Der Transport von Wirkstoffen in die Lungenbereiche kann auf dem Weg
- der inhalativen Applikation und
- der partiellen oder totalen Flüssigkeitsbeatmung erfolgen.

Dabei können
- Wirkstoffe in homogener gelöster Form auf der alveolokapillaren Membran abgesetzt werden,
- Wirkstoffe durch Passage der alveolaren Membran in das durch die Kapillaren fliessende Blut transportiert werden, oder
- Schadstoffe von der Lungenoberfläche abgelöst werden.

Durch Nutzung der sehr großen Austauschfläche kann über diesen Weg die Applikation von Wirkstoffen erfolgen, die auf herkömmlichem Weg, oral oder durch Injektion, schlecht oder gar nicht möglich ist.

Aufgrund der hohen Grenzflächenaktivitäten werden zähflüssig auf der Alveolaroberfläche haftende Schleime aus der Grenzschicht verdrängt, was zur Therapie von Mukoviszidose beiträgt. Auch die Gefahr der Erstickung durch Schleimreste auf den Lungen von Frühgeborenen kann so verringert werden. Weiterhin kann der vorzügliche Spreitungseffekt zur Unterwanderung bzw. zum Vordringen in Lungenemphysembereiche genutzt werden.

Bekanntlich werden staubförmig verteilte Medikamente mittels gängiger Verstäubungsmethoden als Aerosole zur pulmonalen Anwendung gebracht. Auch wenn ein solches Aerosol aus einem leichtflüchtigen PFC als Treibgas besteht, handelt es sich dabei nur um ein heterogenes System, bestehend aus Wirkstoff-Partikeln, PFC-Tröpfchen und ggf. Luft. Denn PFCs besitzen kein Lösevermögen für Kohlenwasserstoffe und deren Derivate, somit auch nicht für Medikamente.

Diese heterogenen Aerosole können bei der oralen Applikation nur zu einem sehr geringen Anteil das Alveolarsystem erreichen, vielmehr werden sie schon vorher in Luftröhre und Bronchien zurückgehalten

### Deponieren von Wirkstoffen auf dem Alveolarsystem durch inhalative oder instillative Applikation

Die erfindungsgemäß beschriebenen Medikamente bestehen aus homogenen, kolloiddispersen Lösungen von Wirkstoffen im Solvens RFRH, wobei die RH-Segmente der SFAs den Kohlenwasserstoff-Gruppierungen der Wirkstoffcluster zugeordnet und die RF-Segmente nach außen gerichtet sind (9). Die so gebildeten, annähernd kugel- bis ellipsenförmigen Mizellen haben eine Größenordnung von 100 nm bis 1 nm.

Die Konzentration des Wirkstoffs ist abhängig von seiner Löslichkeit im SFA und dessen RF/RH-Verhältnis. Je feiner verteilt der zu lösende Stoff, je größer damit seine Oberflächenentwicklung, umso besser ist die Umhüllung mit Solvensmolekeln und somit auch die Löslichkeit.

Um die Wirkstoffe so schnell wie möglich auf der alveolaren Membran abzusetzen, müssen gesättigte, zumindest hochkonzentrierte Wirkstofflösungen appliziert werden. Dadurch wird beim Abatmen des Lösungsmittels, in relativ kurzer Zeit, eine Überschreitung der Löslichkeit des Wirkstoffs und damit dessen Deponieren erreicht.

Je größer die zurückgelassen Molekülcluster sind, umso wahrscheinlicher ist ihr Verbleib auf der Membran.
1. **Inhalative Applikation in Form von Aerosolen**
   Eine Lösung von Wirkstoff im SFA kann verdampft oder mittels Luft, mit Sauerstoff angereicherter Luft oder einem, die Atmung unterhaltenden Gasgemisch rein physikalisch bzw. mechanisch vernebelt werden. Wenn RFRHs mit niedrigem Siedepunkt bzw. hohem Dampfdruck (4, Tabb.1, 2) als Lösemittel eingesetzt werden, wirken diese bereits ab Raum- bis Körpertemperatur als systemeigenes Treibgas.
   Der Anteil aus der Summe von SFA+Wirkstoff und Sauerstoff im Atemgas beträgt etwa 20 bis im Extremfall 100%. Bedingt durch das hohe Spreitungsvermögen der SFAs werden bei der inhalitativen Applikation auch primär geschädigte, insbesondere atelektatische Lungenareale erreicht. Von diesen auf der Alveolarmembran abgelagerten Mizellen werden bei der Atmung die Lösemittelmoleküle, auf Grund ihres bei Körpertemperatur hohen Dampfdruckes, sukzessive exhaliert, während die schwerer flüchtigen Wirkstoffe deponiert bleiben.
**2. Instillative Applikation in Form von Lösungen mittels Partial Liquid Ventilation (PLV)**
   Eine Lösung von Wirkstoff in SFA kann bolusweise mittels Endotrachealtubus oder Bronchoskop appliziert werden.
   Instillativ können größere Wirkstoffmengen verabreicht werden. Auf Grund der größeren Gesamtmenge an applizierter Lösung dauert der Exhalationsprozess des Lösungsmittels länger als bei der Aerosol-Applikation, dagegen können bei der instillativen Applikaktion mehr atelektatische Lungenereale erreicht werden.

Für die inhalativ oder instillativ deponierten Wirkstoffe bestehen drei Pathways:
Sie wirken als Medikament auf der Membran.
Sie werden verschleimt und so wieder ausgeschieden.
Sie passieren die Membran, wenn ihre Partikelgröße < 1 nm beträgt.

Wirkstoffe für die erfindungsgemäße inhalative oder instillative Applikation mit Depotwirkung auf der alveolaren Membran sind Zytostatika, Virostatika, Bakterostatika, Antiasthmatika, Antihistaminika, Proteine, insbesondere Wachstumsfaktoren, Peptide, Vitamine sowie u.a. entzündungshemmende, bronchienerweitemde, durchblutungsfördende Medikamente. Wegen der erforderlichen Löslichkeit im SFA, sind von den jeweiligen Wirkstoffgruppen die Vertreter bevorzugt, deren Molekülstrukturen die geringste, nach außen wirkende, Polarität besitzen.

### Transfer von Wirkstoffen durch die alveokapillare Membran

Außer dem lebensnotwendigen Blutgas-Austausch können bei der Atmung unterschiedliche Spezies über die alveolare Membran in die Blutbahn gelangen. Über die Membran können aber auch Stoffe aus der Blutbahn exhaliert werden.

So ist aus der Blutersatz-Forschung bekannt, dass Perfluorcarbone, die zuvor in emulgierter Form in die Blutbahn gebracht wurden, über die Lunge fast quantitativ abgeatmet werden.

Um erfindungsgemäß Wirkstoffe in die Blutbahn transportieren zu können, müssen diese durch ihre physikalisch-chemischen Eigenschaften, ihre Molekülstruktur und eine im Nanobereich befindliche Partikelgröße von ca. 1 bis 0,1 nm definiert sein. Wie zuvor beschrieben, erfolgt eine Feinverteilung schon bei der Auflösung des Wirkstoffs im SFA.

Durch Einwirkung physikalischer Methoden, wie Ultra-Turrax-Homogenisator, Gaulin-Scherkraft-Homogenisator oder Ultraschall erfolgt eine weitere Verkleinerung, wenn die Wirkstoffe noch als Molekülcluster im Solvens vorliegen. Diese Energiezufuhren sind mit Erwärmen verbunden, deshalb muss während der Homogenisierung gekühlt werden.

Um bei diesen hocheffektiven Homogenisierungsmethoden einen Abbau der chemischen Zusammensetzung der Wirkstoffe auszuschließen, muss das Procedere unter Schutzgas und ständiger Kontrolle durchgeführt werden. Die Umhüllung mit Solvatmolekeln schützt das Substrat vor rückläufiger Koagulation oder Ostwald-Ripening, doch sollten die fertigen Produkte kühl gelagert werden. Erst danach kann die Dosierung mit Sauerstoff erfolgen.

Erstrebenswert ist, dass bereits im Nano-Bereich vorproduzierte, oder ab initio im Solvens SFA in dieser Teilchengröße hergestellte, Wirkstoffe zum Einsatz gelangen.

Ist das Lösungsmittel abgeatmet und sind die Wirkstoffe feinstverteilt auf der Membran deponiert, ist der weitere Transfer-prozess unabhängig davon, ob eine inhalative oder instillative Applikation voranging.

Vorausgesetzt, dass die Solvatmoleküle relativ fest an der äußeren Wirkstoffschicht haften, bedingt durch gute sterische und physikalisch-chemische Wechselwirkung zwischen Solvens und Substrat, können auch Wirkstoffe mit einer Monolayer-Solvathülle abgelagert werden.

Der Transit der Wirkstoffe durch die alveolare Membran und die Aufnahme in die Blutbahn bzw. in das Plasma und der Weitertransport in der Blutbahn, ist ein zeitlich-diffusions-kontrollierter Prozess.

Er wird bestimmt von den physikalisch-chemischen Eigenschaften, der Größe und der räumlichen Struktur der Partikel. Je weniger komplex die Molekülverbände oder Molekeln, je weniger sterische Hinderungen, je weniger polarisierende funktionelle Gruppen, umso leichter ist die Passage. Wie zuvor beschrieben, können sehr kleine, noch mit einer Monolayer von Solvatmolekeln umhüllte Wirkstoffe transferiert werden. Voraussetzung dazu ist die Form und Stabilität dieser Mikromizellen. Diese kleinen Partikel sind mit den Solvatmolekeln so umhüllt, dass deren RF-Gruppen einheitlich nach außen gerichtet stehen. Daraus resultiert eine nicht mehr polarisierbare Gesamtspezies, deren Passage kaum behindert wird.

Die Kinetik des Übergangsprozesses wird somit primär von der Teilchengröße gesteuert. Je molekular-disperser die Partikel sind, umso größer damit ihre Oberflächenentwicklung ist, umso effektiver ist die Wechselwirkung mit den beidseitig an der Membran wirkenden Kapillarkräften und damit der Transfer.

Der erfinderisch beanspruchte intrapulmonale Wirkstofftrans-port mittels semifluorierter Alkane in die Blutbahn ist vorzüglich für die Medikamente geeignet, deren Anwendung sonst mit einer häufigen Infektion, subkutan, intramuskulär oder intravenös verbunden ist. Die intrapulmonale Anwendung ist auch für chronische. Erkrankungen gegeben, deren Behandlung von einer rezidivierenden Medikation mit oral nicht verfügbaren oder oral schlecht wirksamen Medikamenten verbunden ist, oder deren orale Applikation zu starken Nebenwirkungen führt. Auch bietet sich der intrapulmonale Transport für die Wirkstoffe an, die wegen ihrer Zersetzlichkeit den gastrointestinalen Weg nicht überstehen würden.

### Verwendung semifluorierter Alkane zum Transport von Wirkstoffen bei der Totalen Liquid Ventilation (TLV)

Die Verwendung von SFAs zur totalen Flüssigkeitsbeatmung ist in (3-5) patentiert. Erfinderisch neu ist, und wie zuvor für die instillative PLV-Applikation beschrieben, dass auch bei der TLV mit Sauerstoff gesättigte oder teilgesättigte SFAs mit gelösten Wirkstoffen applizierbar sind.

Solche Anwendungen sind vorzüglich dann von Interesse, wenn sehr schnell atelektatische Lungenbereiche, total oder weitgehend total, zu erreichen bzw. zu entfalten und dabei gleichzeitig Wirkstoffe zu applizieren sind.

Dies betrifft insbesondere schleim- und krampflösende, bronchienerweitemde, oberflächenaktive, entzündungshemmende oder antiischämische Substanzen.

Das Ablösen und Entfernen von zähhaftendem Schleim von den Lungen Frühgeborener sowie bei Mukoviszidose, das Entfernen verschleimt-verkapselter, anorganischer und organischer Schadstoffe und Umweltschadstoffe, die Entfernung teeriger Ablagerungen aus den Bronchien und Alveolen, u.a., sind Beispiele für diese Applikationsform.

### Intrapulmonale Verwendung semifluorierter Alkane in Form von wasserhaltigen w/o - oder o/w - Emulsionen als Wlrkstoffträger

In **(3-5)** wird die Verwendung von SFAs zur Herstellung von wasserhaltigen o/woder w/o-Emulsionen mittels biokompatibler Emulgatoren, bei gegebener Gaslöslichkeit, beschrieben.

In solchen Emulsionen sind gleichzeitig auch Wirkstoffe für die Lungenheilkunde löslich. Im Falle der o/w-Emulsionen können die Wirkstoffe, statt in ihrer Basisform, besser in Form der wasserlöstichen Hydrochloride, Phosphate oder Alkalisalze eingesetzt werden.

Somit wird auf Grund der Löslichkeit von Medikamenten und auf Grund der gleichzeitigen Löslichkeit von Atemgasen in diesen Systemen, beschrieben diese Emulsionen in Kombination mit den zuvor für die intrapulmonale Applikation benannten Wirkstoffen für die Lungenheilkunde zu verwenden. Die o/w- oder die w/o-Emulsionen können inhalativ als Aerosole oder instillativ als Flüssigkeiten appliziert werden.

### Beispiel 1

Eine Lösung von 6000 mg Ibuprofen in 1 L F6H8 wurde hitzeste-rilsiert als Bulkware erstellt und zum klinischen Experiment an 7 Schweinen zur Verfügung gestellt.

Die gewonnen Ergebnisse belegen, dass die Applikation einer SFA-Ibuprofen-Lösung mittels intratrachealer Instillation zu einer schnellen systemischen Resorption führt. Bei einem Blutvolumen von ca. 70 ml/kg KG bei Schweinen werden bereits binnen weniger Sekunden, mehr als 55 % des in SFA gelösten Ibuprofens systemisch resorbiert. Toxische systemische Nebenreaktionen traten nicht ein ((10), Interner Forschungsbericht im Anhang).

### Beispiel 2

Eine gesättigte Lösung von 31000 mg alpha-Tocopherol in 1 L F6H8 wurde hitzesterilisiert als Bulkware erstellt. Im klinischen Experiment an Schweinen wurde von dieser Lösung bolusweise intratracheal instilliert. Dabei wurde kein Übertritt des Medikamentes in die Blutbahn festgestellt. Nach Abatmen des SFA verblieb das Medikament im Alveolarbereich.

### Beispiel 3

Eine Lösung von 37000 mg Retinolpalmitat in 1 L F4H6 wurde hitzesterilisiert, als Bulkware erstellt und unter Sterilbedingungen in 50 ml Glasgefäßen deponiert. Der Inhalt dieser Gefäße kann entweder mittels Druckzerstäuber als Aerosol oder mittels Bronchoskop intratracheal appliziert werden.

### Beispiel 4

Eine gesättigte Lösung von 30 mg 5-Fluoruracil in 1 L F6H8 wird durch Sterilfiltration mittels 0,2 Mikrometer-Steril-filter in 20 ml Glasgefässen deponiert. Der Inhalt dieser Gefässe kann, mittels bekannter Vernebelungsmethoden sowie mit den aus der Anästhesie bekannten Vorrichtungen zum Verdampfen flüssiger Narkosemittel, als Aerosol verdampft und inhalativ appliziert werden.

### Beispiel 5

Bromhexin wird in F2H2 bis zur Sättigung bei 23 °C gelöst. Die Lösung wird unter Kühlen mittels 0,2 Mikrometer-Filtern steril filtriert und unter Sterilbedingungen in 20 ml Gefässen, aus Glas oder Aluminium, ausgestattet mit Düse und Druckverschluss, deponiert. Beim Öffnen des Verschlusses versprüht sich die Lösung oberhalb + 23 °C durch den Dampfdruck des Lösungsmittels, als systemeigenes Treibmittel, als Aerosol.

### Beispiel 6

Ibuprofen wird bei 25 °C bis zur Sättigung in 30% v/v F2H3i und 70% F2H3 gelöst. Die Lösung wird steril filtriert und unter Sterilbedingungen in 20 ml Gefässen, aus Glas oder Aluminium, ausgestattet mit Düse und Druckverschluss, gelagert. Beim öffnen des Verschlusses versprüht sich der Inhalt oberhalb + 35 °C, bedingt durch die Dampfdrucke der SFAs, als Aerosol.

### Beispiel 7

Eine Lösung von 120 mg Oseltamivir (Tamiflu^{R}) in 1 L F4H6 wird unter Ausschluss von Atmosphärilien und unter Sterilbedingungen mittels Ultraschall homgenisiert. Anschliessend werden Einheiten zu 20 ml in Glasgefässen bei 133 °C hitze-steriliert.

### Referenz beispiel 8

Zur Präparation einer w/o-Ernulsion werden 5% v/v Wasser, 0,02 w/v Ambroxol-HCL, 5,0 w/v Eidotterlipid und 95% v/v F6H8 mittels Hochdruck-Homogenisator unter Kühlung homogenisiert. Danach wird die opake Emulsion steril filtriert und in 10 ml-Einheiten bei + 5 °C gelagert. Die Emulsion kann, mittels Ultraschall versprüht als Aerosol, inhalativ oder als Flüssigkeit, instillativ appliziert werden.

### Literatur

(1) L.C. Clark, F. Gollan: Science 152 (1966) 1755
(2) C.L. Leach et al: Crit Care Med. 21 (1993) 1270
(3) H. Meinert: WO 97/12852 (1997)
(4) H. Meinert: EP 0 965 334 B1
(5) H. Meinert: US 6,486,212 (2002).
(6) L.A. Dellamary et al.: US 7,205,343 B2 (2007)
(7) M.P. Krafft et al.: WO/2005/099718 (2005)
(8) H. Meinert, T. Roy: Eur.J.Ophthalmol. 10 (2000) 189
(9) Y.K. Kirnet al.: Eur.J.Ophthalmol. 15 (2005) 627
(10) R.Kuhlen, R.Benzberg: Forschungsbericht GB-FM 372037, Klinikum RWTH Aachen/Fa Novaliq Heidelberg

**Tabelle (1)**

| **Beispiele für Semifluorierte Alkane des Typs RFRH und deren Isomere mit Siedepunkten (° C):** | | |
|---|---|---|
| | | |
| Verbindung | Abkürzung | Kp °C |
| CF3CF2CH2CH3 | F2H2 | 23 |
| | | |
| CF₃CF2(CH₂)2CH3 | F2H3 | 60 |
| CF₃CF2(CH₂)7CH3 | F2H8 | 159 |
| CF₃(CF₂)2CH2CH3 | F3H2 | 41 |
| CF₃(CF2)2(CH₂)2CH3 | F3H3 | 65 |
| CF₃(CF₂)2(CH2)7CH3 | F3H8 | 172 |
| CF₃(CF₂)3CH₂CH3 | F4H2 | 67 |
| CF₃(CF₂)3(CH2)3CH3 | F4H4 | 129 |
| CF₃(CF2)3(CH₂)4CH3 | F4H5 | 134 |
| CF₃(CF2)3<CH₂)5CH3 | F4H6 | 163 |
| CF₃(CF2)3(CH₂)6CH3 | F4H7 | 184 |
| CF₃(CF2)3(CH₂)7CH₃ | F4H8 | 196 |
| CF₃(CF2)5CH₂CH3 | F6H2 | 123 |
| CF₃(CF2)5(CH₂)3CH3 | F6H4 | 157 |
| CF3(CF₂)5(CH₂)5CH3 | F6H6 | 187 |
| CF₃(CF2)5(CH₂)7CH3 | F6H8 | 223 |
| CF₃(CF2)5(CH2)iiCH₃ | F6H12 | 290 |
| | | |
| CF3CF₂CH(CH₃)2 | F2H3i | 36 |
| (CF3)₂CFCH₂CH3 | F3iH2 | 38 |
| CF₃(CF2)2CH(CH₃)2 | F3H3i | 60 |
| (CF₃)₂CFCH(CH3)2 | F3iH3i | 64 |

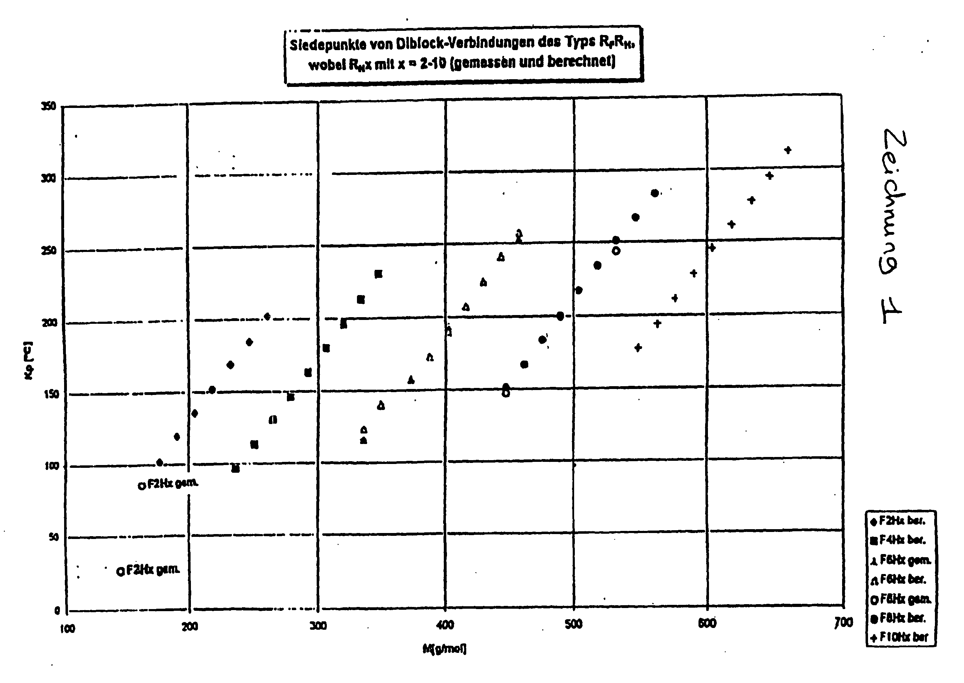

## Patentansprüche

1. Medizinische Zusammensetzung zur Vervendung in der Therapie bestehend aus eine Lösung zum direkten Transport wenigstens eines Medikaments in Lungenbereiche eines Patienten enthaltend ein Trägermittel und mindestens einen Wirkstoff, wobei das Trägermittel wenigstens ein semifluoriertes Alkan des Typs RFRH ist, wobei RF eine lineare oder verzweigte Perfluoralkylgruppe und RH eine lineare oder verzweigte gesättigte Kohlenwasserstoffgruppe ist, wobei die Länge der RF- und RH-Segmente jeweils 2 bis 20 ist, wobei der mindestens eine Wirkstoff in dem Trägermittel kolloid- bis molekulardispers gelöst ist in einer solchen Konzentration, dass er beim Abatmen des semifluorierten Alkans abgelagert wird; wobei
der mindestens eine Wirkstoff ausgewählt ist aus der Gruppe schleim- und krampflöserider, bronchienerweiternder, oberflächenaktiver, entzündungshemmender oder antiischämischer Substanzen; oder wodei
der wenigstens eine Wirkstoff ausgewählt ist aus der Gruppe der Cytostatika, Virostätika, Bakterostatika, Antiasthmatika, Antihistaminika, entzündungshemmenden, bronchienerweiternden, durchblutungsfördemden Medikamente, Proteine, insbesondere Wachstumsfaktoren, Peptide, Vitamine.

2. Zusammensetzung nach Anspruch 1, **dadurch gekennzeichnet, dass** der Wirkstoff im semifluorierten Alkan gesättigt oder zumindest hochkonzentriert gelöst ist.

3. Zusammensetzung nach Anspruch 1 oder Anspruch 2, **dadurch gekennzeichnet, dass** die aus Wirkstoff und semifluoriertem Alkan bestehende Lösung in Form von Micellen vorliegt.

4. Zusammensetzung nach einem der vorhergehenden Ansprüche in einer zur Applikation durch Inhalation als Aerosol oder zur Applikation durch Instillation geeigneten Form.

5. Zusammensetzung nach einem der vorhergehenden Ansprüche, **dadurch gekennzeichnet, dass** sie in einer Form für eine inhalative Applikation vorliegt.

6. Zusammensetzung nach einem der vorhergehenden Ansprüche zur Verwendung zum Wirkstofftransport in ateletaktische Lungenbereiche.

7. Zusammensetzung nach einem der vorhergehenden Ansprüche, **dadurch gekennzeichnet, dass** der enthaltene Wirkstoff eine Partikelgröße im Bereich von 100 nm bis 1 nm hat.

8. Zusammensetzung nach Anspruch 1, **dadurch gekennzeichnet, dass** die Wirkstoffpartikel mit Solvatmolekeln, insbesondere mit einer Monolayer-Solvathülle umhüllt sind.

9. Medizinische Zusammensetzung zur Verwendung in der Therapie von Mukoviszidose bestehend aus eine Lösung zum direkten Transport Wenigstens eines Medikaments in Lungenbereiche eines Patienten enthaltend ein Trägermittel und mindestens einen Wirkstoff, wobei das Trägermittel wenigstens ein semifluoriertes Alkan des Typs RFRH ist, wobei RF eine lineare oder verzweigte Perfluoralkylgruppe und RH eine lineare oder verzweigte gesättigte Kohlenwasserstoffgruppe ist, wobei die Länge der RF- und RH-Segmente jeweils 2 bis 20 ist, wobei der mindestens eine Wirkstoff in dem Trägermittel kolloid- bis molekulardispers gelöst ist in einer solchen Konzentration, dass er beim Abatmen des semifluorierten Alkans abgelagert wird.

10. Zusammensetzung nach einem der vorhergehenden Ansprüche, **dadurch gekennzeichnet, dass** der Wirkstoff eine organische Substanz ist.

11. Zusammensetzung nach einem der Ansprüche 1 bis 7 zum Vordringen in Lungenemphysembereiche.

## Claims

1. Medicinal composition for use in therapy, consisting of a solution for transporting at least one medicament directly into lung regions of a patient, comprising a carrier and at least one active ingredient, wherein the carrier is at least one semifluorinated alkane of the type RFRH, wherein RF is a linear or branched perfluoroalkyl group and RH is a linear or branched saturated hydrocarbon group, wherein the length of the RF and RH segments is in each case from 2 to 20, wherein the at least one active ingredient is dissolved in colloidal to molecularly dispersed form in the carrier in a concentration such that it is deposited when the semifluorinated alkane is eliminated by respiration, wherein the at least one active ingredient is selected from the group mucolytic and antispasmodic, bronchodilatory, surface-active, antiinflammatory or antiischaemic substances, or wherein the at least one active ingredient is selected from the group of the cytostatic agents, antiviral agents, bacteriostatic agents, antiasthmatic agents, antihistamines, antiinflammatory, bronchodilatory, circulation-enhancing medicaments, proteins, in particular growth factors, peptides, vitamins.

2. Composition according to claim 1, **characterised in that** the active ingredient is dissolved in saturated or at least highly concentrated form in the semifluorinated alkane.

3. Composition according to claim 1 or claim 2, **characterised in that** the solution consisting of active ingredient and semifluorinated alkane is in the form of micelles.

4. Composition according to any one of the preceding claims in a form suitable for administration by inhalation as an aerosol or for administration by instillation.

5. Composition according to any one of the preceding claims, **characterised in that** it is in a form for inhalative administration.

6. Composition according to any one of the preceding claims for use for transporting active ingredient into ateletactic lung regions.

7. Composition according to any one of the preceding claims, **characterised in that** the active ingredient it contains has a particle size in the range from 100 nm to 1 nm.

8. Composition according to claim 1, **characterised in that** the active ingredient particles are coated with solvate molecules, in particular with a monolayer solvate shell.

9. Medicinal composition for use in the therapy of cystic fibrosis, consisting of a solution for transporting at least one medicament directly into lung regions of a patient, containing a carrier and at least one active ingredient, wherein the carrier is at least one semifluorinated alkane of the type RFRH, wherein RF is a linear or branched perfluoroalkyl group and RH is a linear or branched saturated hydrocarbon group, wherein the length of the RF and RH segments is in each case from 2 to 20, wherein the at least one active ingredient is dissolved in colloidal to molecularly dispersed form in the carrier in a concentration such that it is deposited when the semifluorinated alkane is eliminated by respiration.

10. Composition according to any one of the preceding claims, **characterised in that** the active ingredient is an organic substance.

11. Composition according to any one of claims 1 to 7 for penetrating pulmonary emphysema regions.

## Revendications

1. Composition médicale destinée à une utilisation en thérapie, composée d'une solution permettant le transport direct d'au moins un médicament dans des zones pulmonaires d'un patient, contenant un vecteur et au moins un principe actif, dans laquelle le vecteur est au moins un alcane semifluoré du type RFRH, où RF est un groupe perfluoroalkyle linéaire ou ramifié et RH est un groupe hydrocarbure saturé linéaire ou ramifié, où la longueur des segments RF et RH est respectivement de 2 à 20, où le principe actif, au moins au nombre de un, est dissous dans le vecteur par dispersion colloïdale à moléculaire dans une concentration telle qu'il est déposé lors de la respiration de l'alcane semifluoré ; dans laquelle le principe actif, au moins au nombre de un, est sélectionné dans le groupe composé de substances mucolytiques et antispasmodiques, bronchodilatatrices, tensioactives, anti-inflammatoires ou anti-ischémiques ; ou dans laquelle le principe actif, au moins au nombre de un, est sélectionné dans le groupe composé des cytostatiques, des virostatiques, des bactériostatiques, des antiasthmatiques, des antihistaminiques, des médicaments anti-inflammatoires, bronchodilatateurs ou stimulant la circulation sanguine, des protéines, en particulier des facteurs de croissance, des peptides et des vitamines.

2. Composition selon la revendication 1, **caractérisée en ce que** le principe actif est dissout de façon saturée ou au moins très concentrée dans l'alcane semi-fluoré.

3. Composition selon la revendication 1 ou la revendication 2, **caractérisée en ce que** la solution composée du principe actif et de l'alcane semi-fluoré se présente sous la forme de micelles.

4. Composition selon l'une des revendications précédentes, dans une forme appropriée pour une application par inhalation en tant qu'aérosol ou pour une application par instillation.

5. Composition selon l'une des revendications précédentes, **caractérisée en ce qu'**elle se présente sous une forme destinée à une application par inhalation.

6. Composition selon l'une des revendications précédentes, pour une utilisation permettant le transport de principe actif dans des zones pulmonaires atélectasiques.

7. Composition selon l'une des revendications précédentes, **caractérisée en ce que** le principe actif contenu possède une taille de particules dans la gamme de 100 nm à 1 nm.

8. Composition selon la revendication 1, **caractérisée en ce que** les particules de principe actif sont enveloppées de molécules de solvate, en particulier d'une enveloppe de solvate monocouche.

9. Composition médicale destinée à une utilisation dans la thérapie de la mucoviscidose, composée d'une solution permettant le transport direct d'au moins un médicament dans les zones pulmonaires d'un patient, contenant un vecteur et au moins un principe actif, où le vecteur est au moins un alcane semi-fluoré du type RFRH, où RF est un groupe perfluoroalkyle linéaire ou ramifié et RH est un groupe hydrocarbure saturé linéaire ou ramifié, où la longueur des segments RF et RH est respectivement de 2 à 20, où le principe actif, au moins au nombre de un, est dissous dans le vecteur par dispersion colloïdale à moléculaire dans une concentration telle qu'il se dépose lors de la respiration de l'alcane semi-fluoré.

10. Composition selon l'une des revendications précédentes, **caractérisée en ce que** le principe actif est une substance organique.

11. Composition selon l'une des revendications 1 à 7, destinée à une pénétration dans des zones d'emphysème pulmonaire.
